(19) Europäisches Patentamt
European Patent Office
Office européen des brevets

(11) Publication number: **0 481 476 A2**

(12) **EUROPEAN PATENT APPLICATION**

(21) Application number: **91117727.7**

(22) Date of filing: **17.10.91**

(51) Int. Cl.[5]: **C07C 69/75**, C07D 303/17, C09D 163/00

(30) Priority: **18.10.90 US 599506**

(43) Date of publication of application: **22.04.92 Bulletin 92/17**

(84) Designated Contracting States: **AT BE CH DE DK ES FR GB GR IT LI LU NL SE**

(71) Applicant: **UNION CARBIDE CHEMICALS AND PLASTICS COMPANY, INC.**
**39 Old Ridgebury Road**
**Danbury Connecticut 06817-0001(US)**

(72) Inventor: **Ashcraft, Arnold Clifton, Jr.**
**R.D. No. 1 Box 355**
**Hightstown (08520), New Jersey(US)**

(74) Representative: **von Hellfeld, Axel, Dipl.-Phys. Dr. et al**
**Wuesthoff & Wuesthoff Patent- und Rechtsanwälte Schweigerstrasse 2**
**W-8000 München 90(DE)**

(54) **Trifunctional unsaturated compounds and derivatives thereof.**

(57) Novel trifunctional unsaturated compounds and mixtures thereof and their derivatives as well as a method for preparing the unsaturated compounds from unsaturated anhydrides and unsaturated alcohols. The unsaturated compounds are used to prepare novel cycloaliphatic epoxide compositions that have utility as coatings, inks, sealants, and adhesives that can be cured under ambient conditions, by thermal means, and by radiation technology.

EP 0 481 476 A2

## Field of the Invention

Difunctional cycloaliphatic epoxides are well known in industry. For many years 3,4-epoxycyclohexylmethane 3,4-epoxycyclohexanecarboxylate has been widely used because of its ready availability, good weatherability, and reasonable reactivity. The good weatherability arises from its lack of aromaticity, and the reasonable reactivity arises from the two cycloaliphatic epoxide groups in its structure and the nature of the hydroxyl groups that form when then epoxide groups undergo ring opening as when they are attacked by an active hydrogen from a Bronsted or other protonic acid. Although this compound has become an important ingredient in ultraviolet light-curable formulations as detailed in U.S. Patents 4, 814,361, 4,818,776, 4,874,798, and 4,892,894 as well as elsewhere, it has not found utility, for example, in room temperature curable coatings not activated by ultraviolet light.

Because of the importance of cycloaliphatic epoxides in radiation-curable formulations and in other high solids, low-energy or low temperature-curing formulations, there has been a need for cycloaliphatic epoxides with functionalities greater than two. Such high functionalities offer cure rates and higher productivity because fewer reactions are required to cause initial gelation and attainment of a tack-free state that allows a coated object to be handled, subjected to further manufacturing operations, or packaged.

Increased cure rates and productivity not only offer economic advantages, they conserve important energy resources and aid in protecting the environment, since there is little opportunity for coating materials to enter the biosphere through vaporization into the atmosphere or through solublization into the waterways. Ideally one would like to have coating cure under ambient conditions with no solvent release.

## Description of the Prior Art

Trifunctional cycloaliphatic epoxides have been described in the literature. For example, U.S. Patent 2,857,402 mentions the reaction of trihydroxyl-functional organic compounds such as 1,2,3-propane triol, trimethylolmethane, 1,1,1-trimethylolethane, 1,1,1-trimethylolpropane, 1,2,6-hexanetriol, cycloaliphtic triols, and aromatic triols such as 1-allyloxy-2,4,6-trimethylolbenzene, 1-phenylpropane-1,2,3-triol, 1,4-bezopyran-3,5,7-triol and the like with 3-cyclohexenecarbox-ylic acid. Further, it exemplifies the epoxidation of two such reaction products to form 1,1,1-trimethylolpropane tris(3,4-epoxycyclohexanecarboxylate) and 1,2,3-propanetriol tris(3,4-epoxycyclohexanecarboxylate). In one instance, the first mentioned triepoxide was reacted with sulfuric acid for long times at elevated temperatures.

However, not previously suggested or disclosed are the novel unsaturated compounds, their novel epoxidized derivatives, their useful range of properties, or the process for their manufacture.

## SUMMARY OF THE INVENTION

The novel trifunctional unsaturated compositions and their derivatives of this invention are those prepared from certain unsaturated anhydrides and unsaturated alcohols. The products are formed by a combination of an addition reaction in which a hydroxyl group reacts with an anhydride group, a condensation reaction in which a carboxyl group reacts with a hydroxyl group to form triene products. The triene compositions are useful as intermediates for the manufactures of cycloaliphatic epoxide compositions that have utility as coatings, inks, sealants, and adhesives. The epoxides of this invention can be used alone or formulated with other cycloaliphatic epoxides, glycidyl epoxides, polyols, polycarboxylic acids, vinyl ethers, and acrylates to form compositions that can be cured at room temperature without added catalyst or more rapidly at elevated temperatures with catalysts. In addition, if formulated with cationic photoinitiators such as the aryl sulfonium or iodonium hexafluorophosphates or hexafluoroantimonates, ultraviolet light-curable coatings and inks can be prepared.

The coating, ink, sealant, or adhesive compositions of this invention can be used for both decorative and functional purposes. The end uses include coatings and/or inks for metal, glass, plastics, wood, paper, and paperboard substrates. The coatings and/or inks can be used as electrodepositable automobile primers, can coatings, furniture coatings, automobile topcoats, decorative coatings, protective coatings for plastics to impart mar or scratch resistance, overprint varnishes for various substrates; electrical or electronic coatings such as photoresists, solder masks, and conformal coatings; inks for written or pictorial communication, sealants for dual-in-place switches or other end uses, and the like. Coatings containing the compounds of this invention can be applied by a variety of techniques, such as spray or roll coating, and the like, and can be cured by a variety of technques, including actinic radiation, exposure to thermal energy, and the like.

## DESCRIPTION OF THE INVENTION

The Trifunctional Unsaturated Compounds

The novel trifunctional compounds of this invention are those defined by the following structural formulas. The novel trifunctional unsaturated compounds are defined by average structural formula:

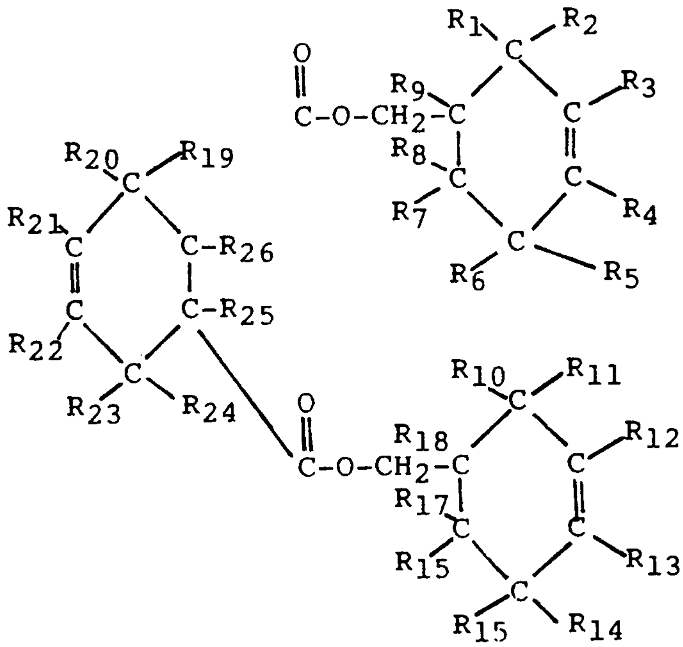

I

wherein $R_1$ to $R_{26}$ are the same or different and can be hydrogen, phenyl, or unsubstituted or substituted alkyl or cycloalkyl groups of one to about 8 carbon atoms, preferably one or two carbon atoms, provided only that the substitutes do not significantly interfere with the use of compositions for its intended purpose.

The novel trifunctional cycloaliphatic epoxide compounds are defined by average structural formula II:

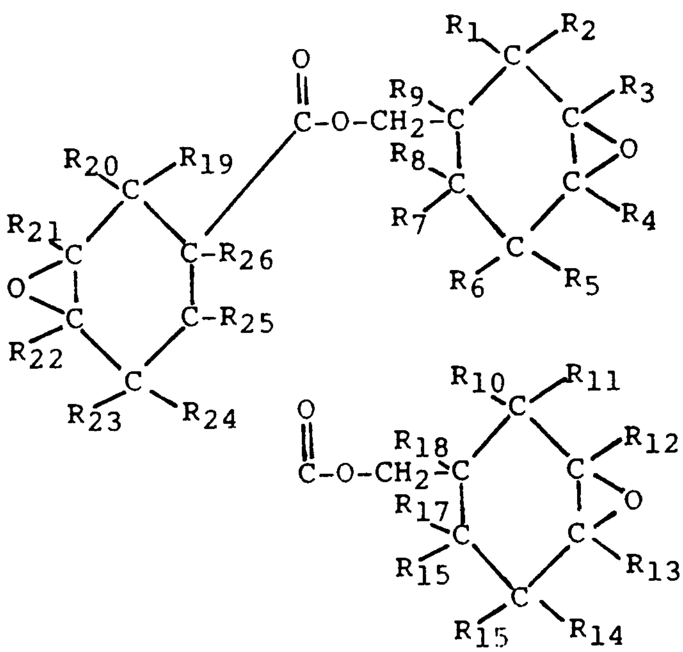

II

wherein $R_1$ through $R_{26}$ are as defined above.

The trifunctional unsaturated compounds of the invention are produced by reaction of an unsaturated anhydride of the average structural formula III:

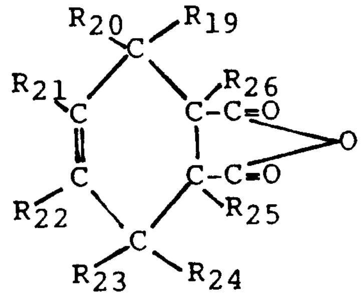

III

with an unsaturated alcohol of average structural formula IV and/or V:

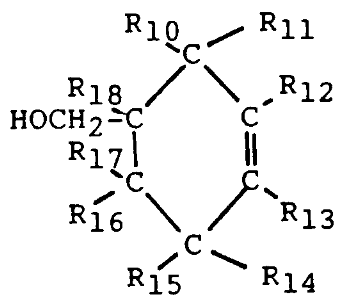

IV

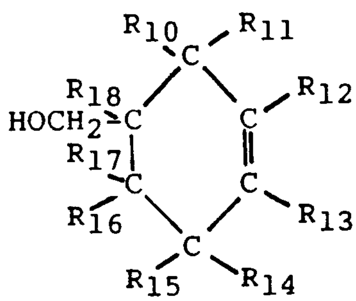

V

Although this invention is not intended to be limited to any particular reaction mechanism, it is thought that the products of the invention are formed in the following manner, wherein $R_1$ through $R_{26}$ are hydrogen. First, anhydride molecules react with alcohol molecules in an anhydride ring-opening step to make an unsaturated, carboxylic acid-functional intermediate of structural formula VI.

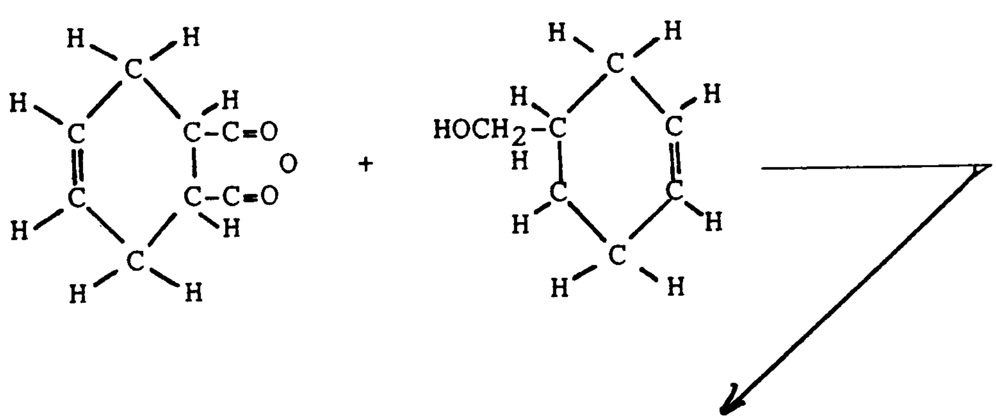

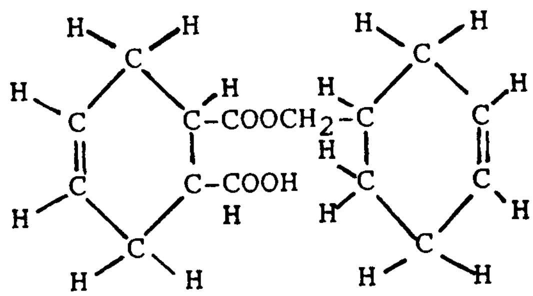

VI

Compound VI then reacts with another molecule of alcohol through a condensation reaction which involves loss of a water molecule to form the novel trifunctional unsaturated compounds, Formula I, of the invention.

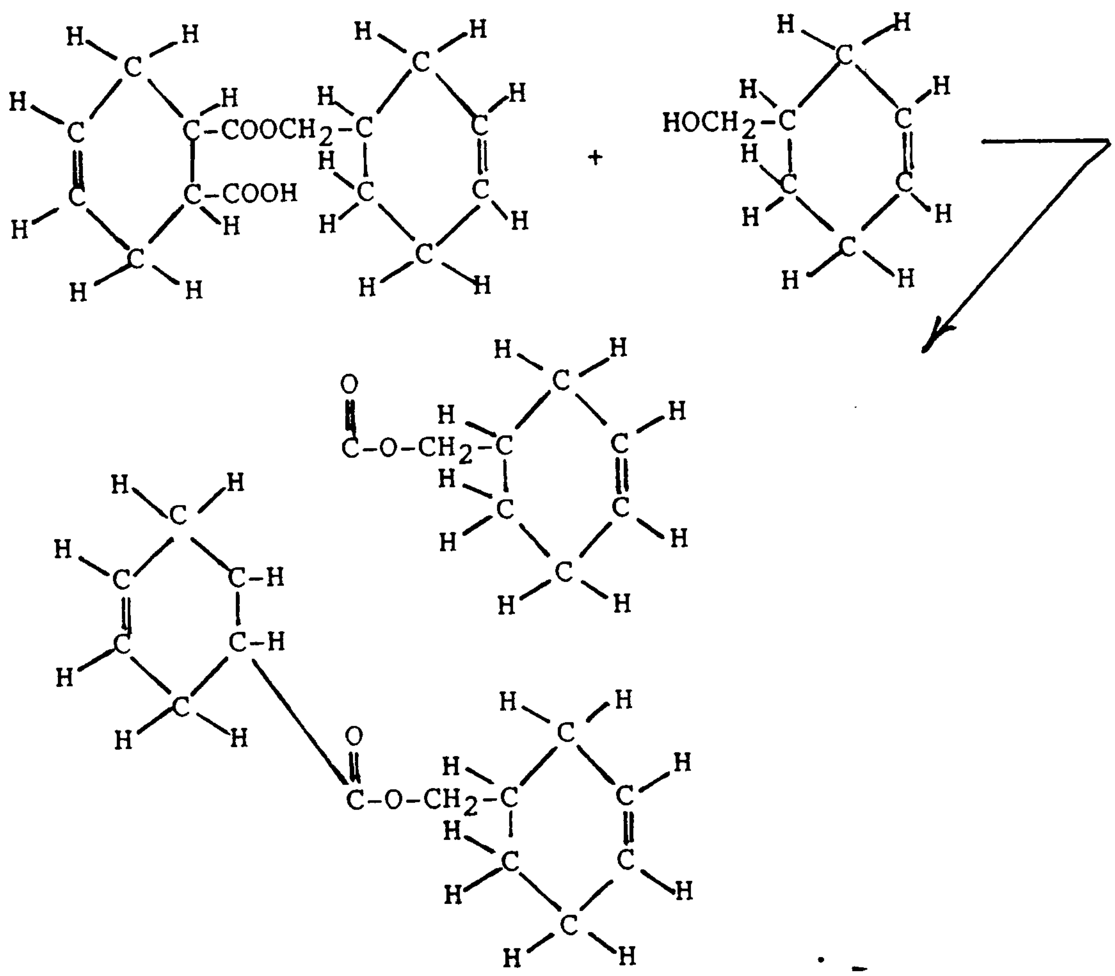

It will be understood by those skilled in the art that when different unsaturated alcohols are used, correspondingly different products will be obtained. In addition, since the anhydride will react faster with a hydroxyl group than will a carboxyl group with a hydroxyl group, different unsaturated alcohols can be fed in sequences, if desired, to cause one type product to produced over another type product. Although it is less preferred, it will be further understood by those skilled in the art that dicarboxylic acids of the structural formula VII:

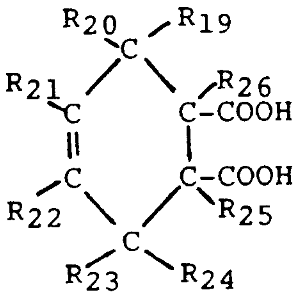

VII

can be used alone or as a replacement for a portion of the unsaturated anhydride to form the products of the invention. When VII alone is used, it will be understood that only condensation reactions are involved in forming the products.

The unsaturated alcohols IV and V suitable for use in production of the trifunctional unsaturated compounds of this invention include tetrahydrobenzyl alcohol (also known as cyclohexene-1-methanol),

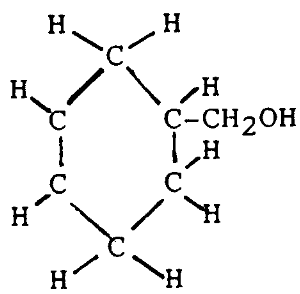

,

6-methyl-3-cyclohexene-1-methanol,

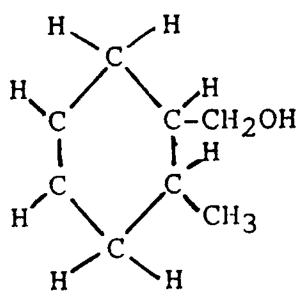

,

3-methyl-3-cyclohexene-1-methanol,
4-methyl-3-cyclohexene-1-methanol,
4,6-dimethyl-3-cyclohexene-1-methanol,
2-butyl-3-cyclohexene-1-methanol,
6-ethyl-3-cyclohexene-1-methanol,
1-methyl-3-cyclohexene-1-methanol,
1-ethyl-3-cyclohexene-1-methanol,
3,6-dimethyl-3-cyclohexene-1-methanol,
4,6-dimethyl-3-cyclohexene-1-methanol,
3-phenyl-3-cyclohexene-1-methanol,
6-phenyl-3-cyclohexene-1-methanol,
2,5-dimethyl-2-phenyl-3-cyclohexene-1-methanol, and the like. The preferred unsaturated alcohols are
3-cyclohexene-1-methanol and
6-methyl-3-cyclohexene-1-methanol.

The unsaturated anhydrides, III, suitable for use in production of the trifunctional unsaturated compositions of this invention include 1,2,3,6-tetrahydrophthalic anhydride,

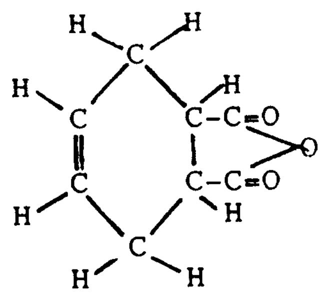 ,

cis-1,2,3,6-tetrahydrophthalic anhydride, mixed-methyl-cis-1,2,3,6-tetrahydrophthalic anhydride, 4-methyl-1,2,3,6-tetrahydrophthalic anhydride,

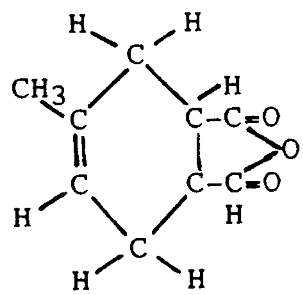 ,

5-methyl-1,2,3,6-tetrahydrophthalic anhydride, and the like. As previously mentioned, the dicarboxylic acids corresponding to these anhydrides can also be used alone or in combination with anhydrides to prepare the unsaturated compositions of the invention.

In a particular embodiment of this invention, unsaturated anhydrides such as 1-cyclopentene-1,2-dicarboxylic anhydride,

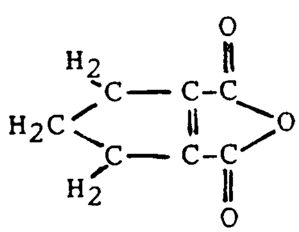 ,

maleic anhydride, and 3,4,5,6-tetrahydrophthalic anhydride,

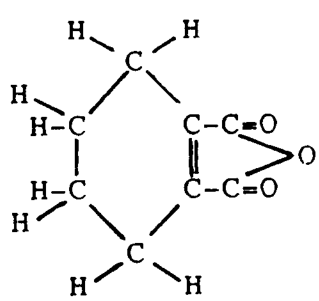 ,

and the like, can be used to prepare novel compounds by reaction with tetrahydrobenzyl alcohol.

Catalysts for the reaction are those used for condensation reactions and include metallic oxalates such as stannous oxalate, zinc oxalate, and the like; metallic alkanoates such as stannous octanoate, zinc octanoate, dibutyltin dilaurate, and the like; sodium aluminate, mixtures of sodium acetate and aluminum acetate sodium bisulfate, and the like; organic titanates such as triisopropyl titanate, butyl titanate, and the

like. Preferred catalysts include stannous oxalate and stannous octanoate.

The reaction can be carried out at temperatures of from about 100°C to about 300°C, preferably from about 110°C to about 250°C. The reaction may be carried out at atmospheric pressure, although higher or lower pressures can be used. It is preferred to carry out the reaction at atmospheric or subatmospheric pressure to facilitate water-of-condensation removal. The reaction is carried out for a period of from about 2 to 72 or more hours, with it understood that the length of reaction time is dependent on the reactants used, the ratio of the reactants, the catalyst, and the temperature of the reaction.

A solvent suitable for azeotoping of the water of condensation may be added to the reaction mixture. Such solvents include ethyl benzene, xylene, as well as other organic solvents known to those skilled in the art of condensation reactions.

The color of the product can, when desired and/or when necessary, be markedly improved by passing a solution of the trifunctional unsaturated compound through a bed of, for example, activated charcoal covered with activated alumina. Suitable solvents are heptane, mixtures of heptane and ethyl acetate, and the like.

## The Trifunctional Cycloaliphatic Epoxides

The novel trifunctional cycloaliphatic epoxide compounds of this invention, II, are prepared by reaction of the trifunctional unsaturated compounds, I, of the invention with an epoxidizing agent such as peracetic acid. For example:

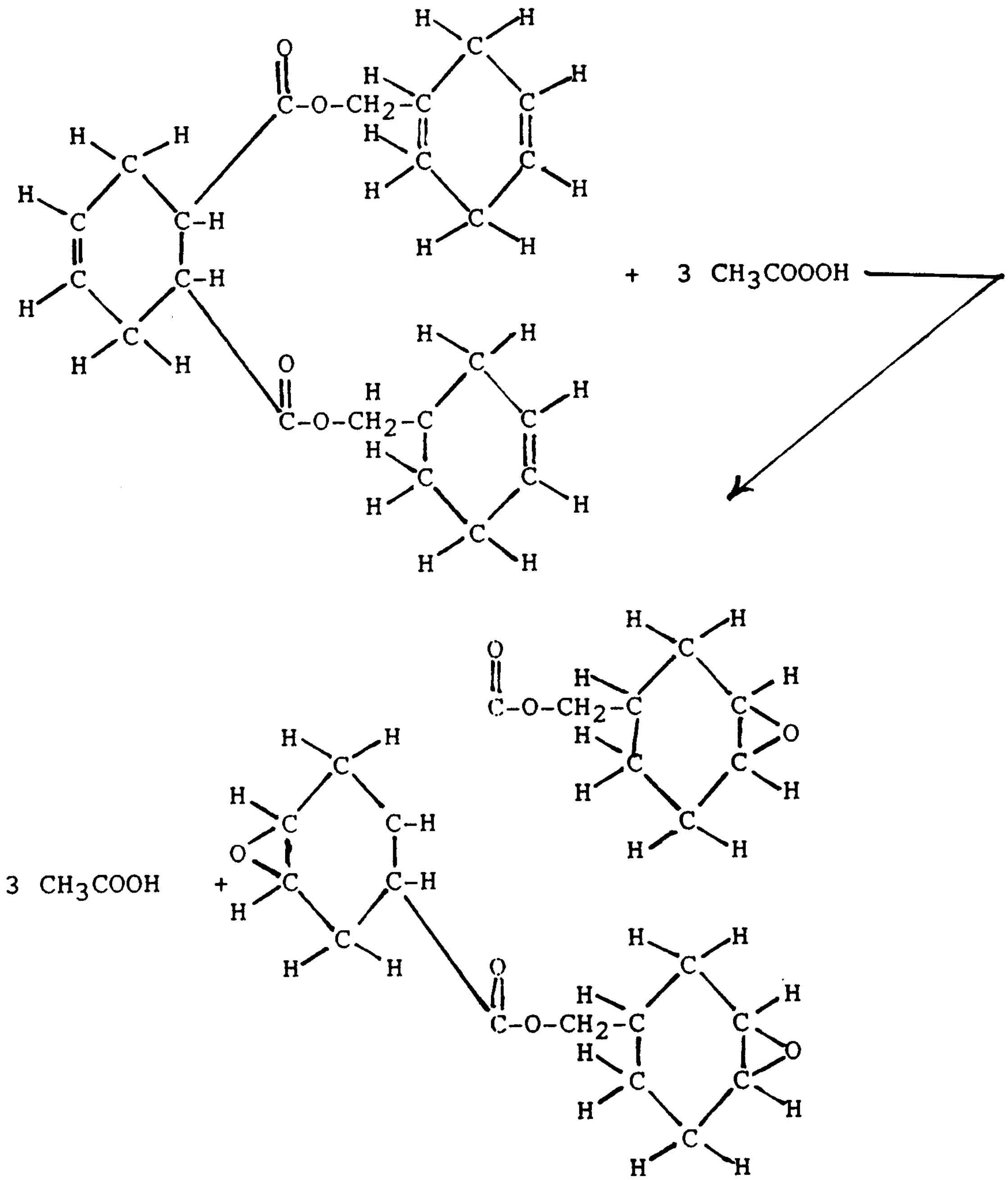


The oxidation and formation of epoxides is readily accomplished by those skilled in the art. Useful methods of epoxidation are described in U. S. Patent Nos. 2,716,123, 2,745,847, and 2,750,395, in H. Lee and K. Neville, "Handbook of Epoxy Resins," McGraw-Hill Book Co., NY (1967), C. A May and Y. Tanaka, "Epoxy Resins Chemistry and Technology," Marcel Dekker, Inc., NY (1973), as well as elsewhere in the literature.

Epoxidizing agents of various types can be used. These agents can be formed in situ from hydrogen peroxide and an organic acid such as acetic acid, can be preformed and used as a peracid, or can be in the form of a dioxirane such as dimethyldioxirane, and the like. Illustrative of the peracids that can be used in carrying out epoxidations are perbenzoic acid, peracetic acid, perpropionic acid,, percaproic acid, permonochloroacetic acid, perbutyric acid, perlactic acid, permonosuccinic acid, t-butylperbenzoic acid, and the like. When used, the peracids are usually dissolved in a solvent such as ethyl acetate to minimize explosive and other hazards.

The trifunctional unsaturated compounds are reacted with the epoxidizing agent at temperatures of less than about 5 °C to about 90 °C, preferably at temperatures of about 10 °C to 60 °C, and most preferably at temperatures of 20 to 50 °C. The time required for reaction will vary depending upon the particular reactants charged and the temperature, facts which are well known to those skilled in the art of epoxidation

chemistry. In general, the peracid solution is carefully and very slowly added to the reactor containing the unsaturated polylactone acrylate, in either a neat form or preferably dissolved in a suitable inert solvent such as ethyl acetate, which is held at a relatively constant reaction temperature. Rate of peracid addition should be such that a desired maximum temperature is not exceeded. The exothermic oxidation reaction that takes place is controlled by cooling the reaction mass to the desired reaction temperature. Peracid addition rate is decreased or stopped if necessary to maintain temperature control. A method of quenching the reaction is usually made available and maintained as, for example, in the laboratory an ice/water bath is available. The reaction product is then optionally washed one or more times with water to remove by-product acid, such as acetic acid when peracetic acid is used, and oxidizing agent. The product is isolated by vacuum stripping of the organic acid that is formed and the solvent that had been used to dissolve the unsaturated polylactone acrylate and/or peracid acid or other oxidizing agent. Optionally, the product may be washed one or more times with water. If desired, the product may be redissolved and reisolated by vacuum stripping using conventional techniques, distillation, or other recovery method.

Formulated Composition Using Trifunctional Cycloaliphatic Epoxide Compounds of the Invention

The trifunctional cycloaliphatic epoxide compounds can be formulated to produce coating, ink, and sealant compositions by the addition of other reactants, polyols, crosslinking agents, pigments, fillers, surfactants, slip agents, catalyst or other initiators and promoters when desired, and other additives conventionally used in the production of such compositions. The trifunctional cycloaliphatic epoxide compounds can be cured into coatings, adhesives, inks, and sealants by reaction of the epoxide groups with suitable crosslinking agents such as carboxylic acids of various types, polyols, etc. Although this entire invention is not meant to be limited by any particular reaction mechanism, the above mechanism as well as other mechanisms are offered as realistic descriptions of how various reactions occur as the products are made and used.

Although homopolymers can be made from the trifunctional cycloaliphatic epoxide compounds, usually they will be used in combination with other compounds and the final product formed through a copolymerization process. Formulations will contain from about 1 to about 100 weight percent trifunctional cycloaliphatic epoxide compounds, preferably from about 1 to about 50 weight percent trifunctional cycloaliphatic epoxide compounds. For example, trifunctional cycloaliphatic epoxide compounds can be used alone, can be combined with each other, and/or can be combined with other epoxides, polyols, and vinyl ethers to form useful coating films after copolymerization has taken place. The compositions can be used for a broad variety of end uses including automotive coatings, can coatings, general metal coatings, decorative coatings, electronics coatings including solder masks, photoresists, and conformal coatings, protective coatings for compact and optical discs, and the like, as well as inks for printed words or designations, for pictorial representation, and the like, for molded objects such as printing plates, and for sealants used in the automotive, home and electronic industries. Coatings can be applied by various techniques, illustrative of which are spray coating, roll coating of various types, dip coating, electrodeposition, brush, and the like. Particularly useful application methods are spray coating, dip coating, and electrodeposition. The coatings can be cured by a variety of techniques, including radiation, thermal, air drying, and the like, depending on the particular system being formulated.

In producing coatings curable with actinic radiation and preferably ultraviolet light, the trifunctional cycloaliphatic epoxide compounds are combined with other cycloaliphatic epoxides, Novolac epoxides, and the like; vinyl ethers; polyols; onium salt, diazonium salt or other cationic photoinitiators; and, if needed, surfactants. The formulated coatings may contain inert solvents for the purpose of decreasing viscosity and improving application characteristics or inert polymers, fumed silicas, and the like, to thicken the formulated coating and make it useful in screen printing or other operations. The coatings are cured by exposure to ultraviolet light radiation from a medium pressure mercury vapor lamp with radiation between about 220 and 400 nanometers.

Suitable cycloaliphatic epoxides for combination with the trifunctional cycloaliphatic epoxide compounds of this invention are those having an average of one or more epoxide groups per molecule. Preferably the cycloaliphatic epoxide will be a mixture of epoxides that will usually contain a major proportion of cycloaliphatic epoxides with two or more epoxy groups per molecule. These cycloaliphatic epoxides will be present in from about 0 to about 95 weight percent of the formulation and preferably from about 30 to about 60 weight percent of the formulation. Illustrative of suitable cycloaliphatic epoxides are the following:

3,4-epoxycyclohexylmethyl 3,4-epoxycyclohexane- carboxylate having the formula:

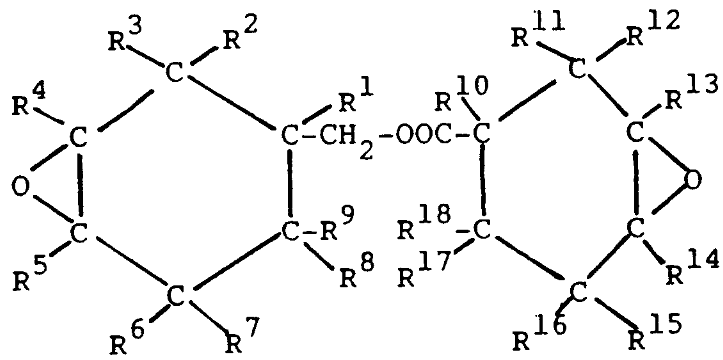

wherein $R^1$ through $R^{18}$, which can be the same or different, are hydrogen or alkyl radicals generally containing one to five carbon atoms inclusive, and preferably containing one to two carbon atoms, as for example methyl, ethyl, n-propyl, i-propyl, n-butyl, s-butyl, i-butyl, t-butyl, and the like. Particularly desirable compounds are those wherein $R^1$ through $R^{18}$ are hydrogen. Among specific compounds falling within the above structure are the following: 3,4-epoxy-cyclohexylmethyl 3,4-epoxycyclohexane-carboxylate; 3,4-epoxy-1-methylcyclohexylmethyl
3,4-epoxy-1-methylcyclohexanecarboxylate;
6-methyl-3,4-epoxycyclohexylmethyl
6-methyl-3,4-epoxycyclohexane-carboxylate;
3,4-epoxy-3-methylcyclohexylmethyl
3,4-epoxy-3-methylcyclohexanecarboxylate;
3,4-epoxy-5-methylcyclohexylmethyl
3,4-epoxy-5-methyl-cyclohexane- carboxylate. Other suitable compounds are described in, for example, U.S. Patent No. 2,890,194;

diepoxides of cycloaliphatic esters of dicarboxylic acids having the formula:

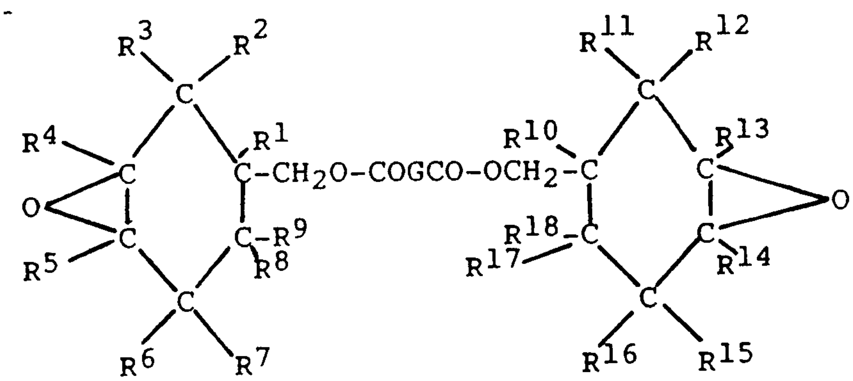

where $R^1$ through $R^{18}$ which can be the same or different are as defined for $R^1$ through $R^{18}$ above; G is a valence bond or a divalent hydrocarbon radical generally containing one to 10 carbon atoms, inclusive, and preferably, containing three to six carbon atoms, inclusive, as for example alkylene radicals, such as trimethylene, methyltrimethylene, tetramethylene, pentamethylene, hexamethylene, 2-ethylhexamethylene, octamethylene, nonamethylene, and the like; cycloaliphatic radicals such as 1,4-cyclohexane, 1,3-cyclohexane, 1,2-cyclohexane, and the like. Particularly desireable epoxides, falling within the scope of this structure, are those wherein $R^1$ through $R^{18}$ are all hydrogen and G is alkylene containing three to six carbon atoms. Among specific diepoxides of cycloaliphatic esters of dicarboxylic acid are
bis(3,4-epoxycyclohexylmethyl)oxylate,
bis(3,4-epoxycyclohexylmethyl)adipate,
bis(3,4-epoxy-6-methylcyclohexylmethyl)adipate,
bis(3,4-epoxycyclohexylmethyl)pimelate, and the like. Other suitable compounds are described in, for example, U. S. Patent No. 2,750,395.

Other useful cycloaliphatic diepoxides include 2-(3,4-epoxycyclohexyl-5,5-spiro-3,4-epoxy)cyclohexane-m-dioxane, halogen or monovalent hydrocarbon variations of this compound, and the like, as further defined

in U.S. Patent No. 3,318,822; cyclopentadiene diepoxide, cyclohexane diepoxide, and the like.

The preferred cycloaliphatic diepoxides are 3,4-epoxycyclohexylmethyl-3,4-epoxycyclohexane carboxylate, bis(3,4-epoxycyclohexylmethyl)adipate or mixtures thereof.

The compositions may include a cycloaliphatic monoepoxide that functions as a reactive diluent and contributes to overall coating properties. This monoepoxide may be an unsubstituted monoepoxide such as cyclohexene oxide or a substituted monoepoxide with alkyl groups of 1 to about 6 carbon atoms, halogen, ester groups, caprolactone groups, alkylene oxide groups, vinyl groups, and the like. Illustrative of the monoepoxides one can mention limonene monoepoxide, 4-vinyl cyclohexene monoepoxide, norbornene monoepoxide, alpha-pinene monoepoxide, cyclohexene monoepoxide; polylactone epoxides with the structure:

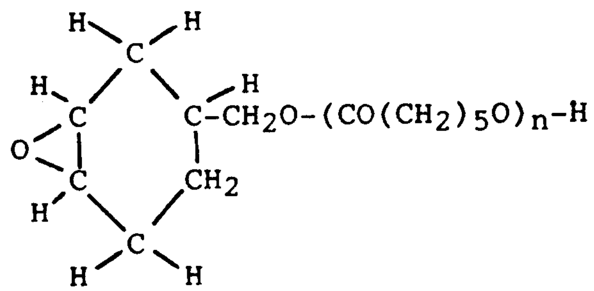

where n is from 1 to about 5, as well as similar epoxides wherein the caprolactone is replaced with methyl caprolactones, valerolactones, enantholactones, propylene oxide, ethylene oxide, or mixtures of these compounds, and the like; 3,4-epoxycyclohexylmethyl acylate and methacrylate, and the like; acrylated and methacrylated polylactone epoxides with the structure:

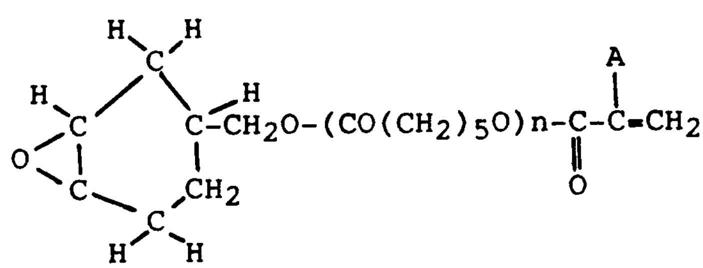

wherein A is either hydrogen or methyl, as well as similar acrylated epoxides wherein the caprolactone is replaced with methyl caprolactones, valerolactones, enantholactones, propylene oxide, ethylene oxide, or mixtures of these compounds, and the like. The cycloaliphatic monoepoxide may be used in the composition in amounts of from 0 to about 50, preferably from 0 to about 30, and most preferably from 0 to about 20 weight percent of the cycloaliphatic epoxide used.

If desired, minor amount of glycidyl epoxides such as the diglycidyl ethers of Bisphenol-A, diglycidyl ethers of brominated Bisphenol-A, cresol-novolac epoxy resins, epoxy phenol novolac resins, diglycidyl ethers of 1,4-butanediol, and the like, can be used in the radiation cure formulations containing the trifunctional cycloaliphatic epoxide compounds of this invention.

Various vinyl ethers can be used in the radiation cure formulations of this invention. Suitable vinyl ethers are illustrated by the dihydropyranyls and di-(dihydropyranyl) compounds as described in U.S. Patent 4,645,781, diethylene glycol vinyl ether, triethylene glycol vinyl ether, and the like, a number of which have been described by J. V. Crivello, J. L. Lee, and D. A. Conion, Proceedings of Radiation Curing IV, Chicago, IL, p 4-28, Sep. 20-23 (1982).

A variety of polyols can be used in the radiation curable formulations containing the trifunctional cycloaliphatic epoxide compounds. Illustrative of these are the mono-, di-, tri-, and higher functionality caprolactone and other lactone polyols; the alkylene oxide polyols including ethylene oxide, propylene oxide, and copolymeric ethylene oxide/propylene oxide polyols, ethylene oxide-capped propylene oxide polyols, alkylene oxide derivatives of esterdiols such as the 4-mole ethoxylate of Esterdiol-204; polymer polyols; polyester polyols, such as hexane diol adipates, butylene adipates, ethylene glycol adipates; poly-(tetramethylene oxide) polyols, polymers with pendant hydroxyl groups such as vinyl chloride/vinyl

acetate/vinyl alcohol terpolymers, styrene/allyl alcohol copolymers, vinyl acetate/vinyl alcohol copolymers, and the like. The polyols are present in from zero to about 60 weight percent of the formulation, preferably from zero to 40 weight percent of the formulation.

When room temperature or other low temperature curable formulations are prepared, polyacids in which a polycaprolactone polyol or an alkoxylated esterdiol such as ethoxylated or propoxylated 2,2-dimethyl-3-hydroxypropyl 2,2-dimethyl-3-hydroxypropionate, is end-capped with an anhydride such as phthalic anhydride or maleic anhydride, are particularly useful. Compounds such as these are described in U. S. Patent Nos. 4,171,423, 4,086,293, 4,086,294, 4,080,318 as well as elsewhere. Other useful polyacids include acrylic copolymers in which acrylic acid or methacrylic acid is copolymerized with for example butyl acrylate, ethyl acrylate, styrene, t-butyl acrylate, and the like; carboxylic acid such as adipic acid, oxalic acid, and the like, as well as other compounds, oligomers, and polymers that contain carboxylic acid functionality. The polyacids are present in the formulations in amounts of from about zero to about 75%, preferably from zero to about 60%.

The photoinitiators which may be used in the radiation-curable formulations containing the trifunctional cycloaliphatic epoxide compounds of this invention typically include one or more of a metal fluoroborate and a complex of boron trifluoride as described in U.S. Patent No. 3,379,653; a bis(perfluoroalkyl-sulfonyl)-methane metal salt, as described in U.S. Patent No. 3,586,616; an aryl diazonium compound as described in U.S. Patent No. 3,708,296; an aromatic onium salt of Group VIa elements as described in U.S. Patent No. 4,058,400; an aromatic onium salt of Group Va elements as described in U.S. Patent No. 4,069,055; a dicarbonyl chelate of a Group IIIa-Va element as described in U.S. Patent No. 4,068,091; a thiopyrylium salt as described in U.S. Patent No. 4,139,655; a Group VIb element in an $MF_6^-$ anion where M is selected from phosphorous, antimony, and arsenic as described in U.S. Patent No. 4,161,478; an arylsulfonium complex salt as described in U.S. Patent No. 4,231,951; an aromatic iodonium complex salt and an aromatic sulfonium complex salt, as described in U.S. Patent 4,256,828; and a bis(4-(diphenylsulfonio)phenyl)sulfide-bis-hexafluorom etallic salts such as the phosphate, arsenate, antimonate, and the like, as described by W. R. Watt and coworkers in J. Polymer Sci.: Polymer Chem. Ed., 22, 1789 (1984). Preferred cationic photoinitiators include the arylsulfonium complex salts, aromatic sulfonium or iodonium salts of halogen-containing complex ions, and aromatic onium salts of Group II, V, and VI elements. Some of such salts are commercially available as FX-512 (3M Co.), UVR-6990 and UVR-6974 (Union Carbide Corp.), UVE-1014 and UVE-1016 (General Electric Co.), KI-85 (Degussa), and SP-150 and SP-170 (Asahi Denka).

The onium salt photoinitiators may be present in the formulations in amounts of from about 0.05 weight percent to about 10 weight percent, preferably from about 0.1 to about 5 weight percent. When photolyzed, these photoinitiators fragment into both cationic species and free radicals, and thus make possible the prospect of dual cure systems such as might be obtained from combinations of cycloaliphatic epoxides and ethylenically unsaturated molecules.

Various acrylates and methacrylates may be included in the formulations if desired. Illustrative of such acrylates are the caprolactone acrylates, trimethylolpropane triacrylate, alkoxylated Esterdiol-204 diacrylates, diethylene glycol diacrylate, 2-ethylhexyl acrylate, neopentyl glycol diacrylate, urethane acrylates, acrylated epoxides or epoxy acrylates, and the like. Acrylates can constitute up to about 35% of the formulation. If desired, one or more additional photoinitiators of the free radical type may be added to the formulation when acrylates are used. Illustrative of such photoinitiators are 2,2-diethoxyacetophenone, acetophenone, alkyl benzoin ethers, 2,2-dimethoxy-2-phenyl acetophenone, and the like. The free radical generators are used in amounts of about 0.5 to about 5 percent, preferably from about 0.5 to about 3 percent of the formulation.

The radiation curable compositions containing the trifunctional cycloaliphatic epoxide compounds of this invention may include additives such as oils; particularly silicone oils, surfactants such as silicone/alkylene oxide copolymers, acrylic polymers such as those available from Monsanto Co. under the MODAFLOWS trademark, silicone oils containing epoxide groups, fluorocarbon surfactants; low molecular weight alcohols, ethoxy alcohols such as butyl CELLOSOLVE (available from Union Carbide), carbitols such as butyl carbitol; and the like, as well as other ingredients known to those skilled in the art of coating formulation. If desired, one may include in the radiation-curable compositions various conventional, non-basic fillers (e.g., silica, talc, glass beads or bubbles, microspheres, aluminum trihydrate, clays, etc.) and other additives such as rubbers, tackifying agents, non-basic pigments, and the like, that will not unduly interfere with the photoinitiated polymerization reactions.

Thermally-curable coatings can be made from the above-described radiation-curable coatings by deleting the above-described photoinitiators from the formulation and substituting a suitable cationic or protonic acid catalyst such as triflic acid salts, boron trifluoride etherate, boron trifluoride, and the like; however, in certain cases of dual radiation-thermal cure systems it may be desireable to use both a

photoinitiator and a catalyst. Particularly well suited catalysts are the triflic acid salts because of the high quality color (i.e., low color) characteristics of these compounds and the shelf-life characteristics, which are dependent on the nature of the salt, of coatings formulated with many of these compounds. Illustrative of these salts are diethylammonium triflate, which is available from 3M Co. as FC-520, triethylammonium triflate, di-isopropylammonium triflate, ethyl,di-isopropylammonium triflate, and the like, many of which are described by R. R. Alm in the October 1980 issue of Modern Coatings. The amount of catalyst used in the thermally-cured formulations is from about 0.05 percent by weight to about 3 percent by weight, preferably from about 0.1 percent by weight to about 1.0 percent by weight, and most preferably from about 0.2 percent by weight to about 0.8 percent by weight of the formulated coating when no pigment or filler is present.

The following examples are illustrative of the present invention and are not intended as a limitation upon the scope thereof. As used in the examples appearing hereinafter, the following designations and terms have the following meanings.

GLOSSARY

Solvent Resistance (Methyl Ethyl Ketone Double Rubs): A measure of the resistance of the cured film to attack by methyl ethyl ketone, in which a film coating surface is rubbed with a methyl ethyl ketone-soaked cloth back and forth with hand pressure. A rub back and forth over the film coating surface with the methyl ethyl ketone- soaked cheesecloth is designated as one "methyl ethyl ketone double rub." Such double rubs are performed until a perforation to the substrate is observed.

Pencil Hardness: Pencil leads of increasing hardness values are forced against the film coating surface in a precisely defined manner as described in ASTM D3363-74 until one pencil lead cuts through the surface of the film coating. The surface hardness is considered as the hardest pencil grade which just fails to cut or mar the film coating surface. The pencil leads in order of softest to hardest are reported as follows: 6B, 5B, 4B, 3B, 2B, B, HB, F, H, 2H, 3H, 4H, 5H, 6H, 7H, 8H, 9H.

| | |
|---|---|
| Polyacid I - | A trifunctional carboxylic acid prepared by reacting three moles of phthalic anhydride with one mole of a 300 molecular weight, trifunctional polycaprolactone polyol, TONE™ 0301, sold by Union Carbide Corporation. |
| Surfactant - | A silicone/alkylene oxide copolymer sold by Union Carbide under the tradename SILWET™ L-7604. |
| FC-520 - | A solution of diethylammoniumtriflate sold by 3M Company. |
| TYZOR™ - | A solution of tetraisopropyl titanate sold by Du Pont Co. |
| Cycloaliphatic | Epoxide-1 - 3,4-epoxycyclohexylmethyl 3,4-epoxycyclohexane carboxylate which is sold by Union Carbide Corp. |
| Cycolaliphatic | Epoxide-2 - bis(3,4-epoxycyclohexylmethyl)adipate which is sold by Union Carbide Corp. |
| Cycloaliphatic | Epoxide-3 - A low viscosity cycloaliphatic epoxide product sold by Union Carbide Corp. as CYRACURE™ UVR-6100. |
| Epoxide-4 - | The diglycidyl ether of Bisphenol A sold by Shell Chemical Company as EPON™ 828. |
| Polyol-1 - | A trifunctional caprolactone polyol having an average molecular weight of 540, an average hydroxyl number of 310, and available from Union Carbide Corp. as TONE™-0305. |
| Polyol-2 - | A difunctional poly(tetramethylene oxide) polyol with an average equivalent weight of 500 and available from Quaker Chemical Co. as POLYMEG™ 1000. |
| Polyol-3 - | A trifunctional propylene oxide polyol having an average equivalent weight of 501, an average hydroxyl number of 112, and commercially available from ARCO Chemical Company. |
| Photoinitiator-1 - | A solution of an aryl sulfonium hexafluoroantimonate salt dissolved in a solvent and is sold by General Electric Co. as UVE-1014 and Union Carbide Corp. as CYRACURE™ UVI-6974. |
| Photoinitiator-2 - | A solution of a aryl sulfonium hexafluorophosphate salt dissolved in a solvent and is sold by General Electric Co. as UVE-1016 and by Union Carbide Corp. as CYRACURE™ UVI-6990 and in a different solvent by 3M Co. as FX-512. |

EXAMPLES

Example 1. Use of sodium aluminate as a catalyst for preparation of bis(tetrahydrobenzyl) tetrahydrophthalate,

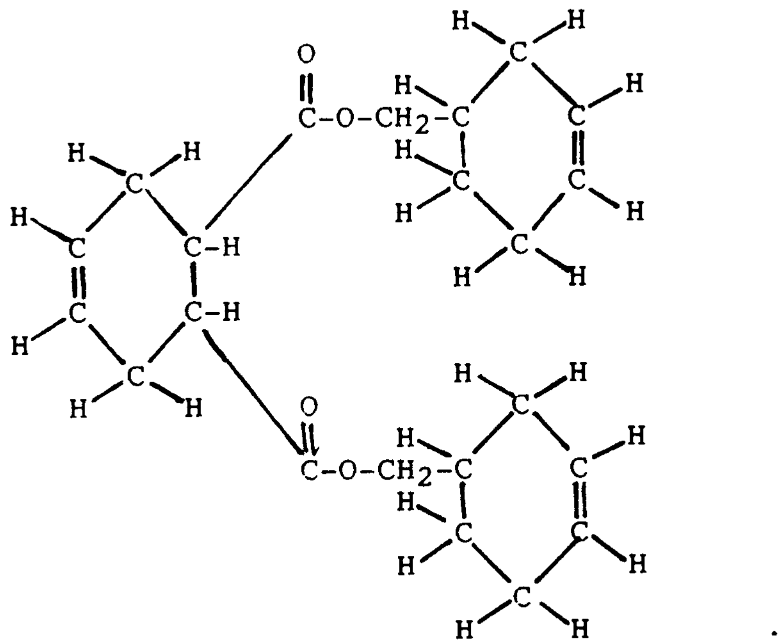

A reactor system comprising a 500-mL, three-necked, glass reaction flask was equipped with a mechanical stirrer, reflux condenser with Dean-Stark trap, addition funnel, and heating mantle. One hundred milliliters of ethyl benzene, an azeotrope solvent, and 76.08 grams (0.50 mole) of 1,2,3,6-tetrahydrophthalic anhydride were added to the reactor. While stirring, 1.9 grams of finely ground sodium aluminate catalyst were added and the slurry was heated to reflux. Then 134.6 grams (1.2 moles) of tetrahydrobenzyl alcohol (3-cyclohexene-1-methanol) were added over an eight minute period at such a rate that a steady reflux was maintained. The reaction was allowed to continue at reflux for about 65 hours. The reaction mass was cooled to room temperature and filtered to yield an amber colored product. The product was then purified by vacuum distillation. Nuclear magnetic resonance studies indicated the triene, comprising bis-(tetrahydrobenzyl) tetrahydrophthalate, had formed.

Example 2. Epoxidation of bis(tetrahydrobenzyl) tetrahydrophthalate to the triepoxide,

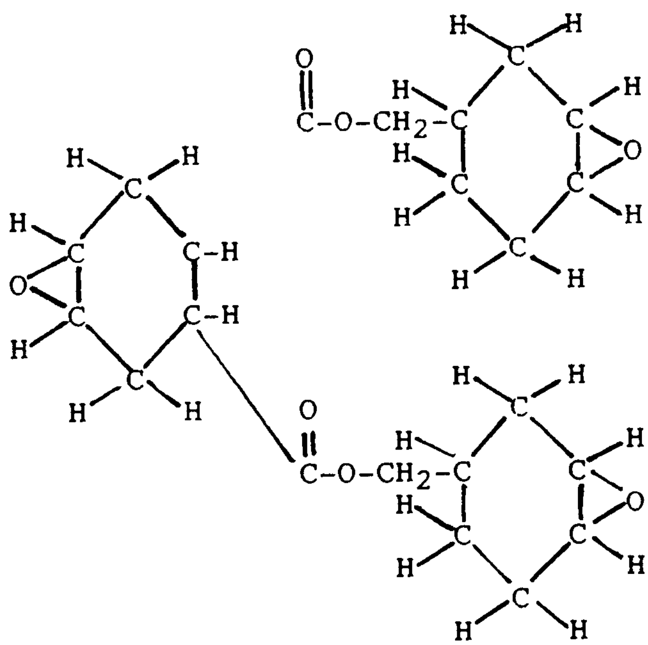

A reactor system comprising a 3-necked, round-bottomed, glass flask equipped with a mechanical stirrer, thermowatch/thermometer, and a y-adapter fitted with an addition funnel and a condenser was mounted over an ice/water bath set on a mechanical jack. Also available was an electric heat gun to aid in temperature regulation. This reactor was charged with 60.22 grams of the bis(tetrahydrobenzyl) tetrahydrophthalate prepared in Example 1, and the temperature was regulated at 45-47° C. Then 176 grams of a 23.09% solution of peracetic acid in ethyl acetate were slowly added over a 35-minute period. The reaction was allowed to proceed at 45-47° C for a period of three hours, after which time analysis indicated that the peracetic acid content was constant and conversion to a product comprising the desired triepoxide was complete. The crude product was purified by passing it through a Pope evaporator using a jacket temperature of 80° C and full laboratory vacuum. The product had an epoxy equivalent weight of 147.

Example 3. Use of TYZOR™ TPT as a catalyst for preparation of bis(tetrahydrobenzyl) tetrahydrophthalate.

The glass reactor system of Example 1 was further equipped with a nitrogen inlet tube and then charged with 76.08 grams (0.50 mole) of 1,2,3,6-tetrahydrophthalic anhydride, 25 mL of ethyl benzene, and 134.6 grams (1.26 moles) of tetrahydrobenzyl alcohol. The mixture was heated with stirring to 220° C and held at this temperature while water of condensation was formed in the Dean-Stark trap. After about an hour, the rate of water formation was slow, and 0.13 gram of TYZOR™ TPT was added. Heating at temperatures of 200-220° C was continued for about 18 hours and analysis indicated the acid number was 0.05. The temperature was reduced and the Dean-Stark trap/condenser was replaced with a distillation head. The ethyl benzene and excess tetrahydrobenzyl alcohol was removed by vacuum distillation at temperatures as high as 158° C and a vacuum of 8 mmHg. When distillation became slow, the reaction mass was cooled to 95° C and a slurry of 0.25 gram of calcium oxide in 5 mL of water was added to deactivate the catalyst and to precipitate salts. Then the vacuum distillation was again begun and continued at temperatures as high as 164° C and 31 mmHg. When distillation ceased, the crude product was cooled to 90° C and 0.2 gram of Celite, a diatomaceous earth filter aid, and 200 mL of heptane were added. The product was filtered to remove salts and Celite and then placed in a Rotoevaporator to remove the heptane. The resulting triene product was clear and had a yellow color.

Example 4. Use of stannous oxalate as a catalyst for preparation of bis(tetrahydrobenzyl) tetrahydrophthalate.

A reactor system comprising a 12-liter, three-necked flask equipped with a nitrogen bubbler-protected reflux condenser and Dehn-Stark trap, heating mantle, thermometer with thermowatch, magnetic stirrer, and septum stopper was assembled. This reactor was charged with 18 moles (2,739 grams) of 1,2,3,6-tetrahydrophthalic anhydride and 43.2 moles (4,846 grams) of tetrahydrobenzyl alcohol. Excess tetrahydrobenzyl alcohol was used to facilitate the reaction. The reactor and its contents were swept with nitrogen. Then, with stirring, the system was heated to reflux with the thermowatch set at 220° C under a nitrogen atmosphere supplied by the bubbler system. Water of condensation was formed and removed by azeotropic distillation with tetrahydrobenzyl alcohol into the Dehn-Stark trap. About one hour and forty-five minutes after the heating was begun, approximately 125 mL of water of condensation had formed. At this time 1.10 grams of stannous oxalate, a catalyst for the condensation reaction, were added. The acid number of the reaction mass was 1.46. The reaction was continued at temperatures of 180-219° C for about 4.75 hours. The reaction mass was allowed to cool to room temperature and stand under ambient conditions overnight. Then the reactor contents were heated to 280° C and after 2.5 hours, the acid number was found to be 0.1005. The condenser and Dehn-Stark trap were removed and replaced with a condenser. Excess tetrahydrobenzyl alcohol and other volatiles were then removed by distillation at a temperature of 219-220° C. The crude, dark-colored product was cooled to room temperature and then decolorized in the following manner.

To a 1000-gram portion of the crude product, 2,000 grams of heptane and 2000 mL of ethyl acetate were added. The solution was then passed through a 1.5-inch glass column that had been first packed with 25 grams of activated charcoal and then 460 grams of activated alumina. The processed product was then placed in a Rotovac evaporator under a temperature sufficient for removal of heptane, ethyl acetate, and any other low-boiling volatiles. This procedure was repeated with 1000-gram or smaller portions until all of the crude product had been processed. A yield of 8,865.42 grams of the expected triene, bis-(tetrahydrobenzyl) tetrahydrophthalate, with a platinum-cobalt color of 50-70 was obtained and stored for future use.

Example 5. Use of stannous octanoate as a catalyst for preparation of bis(tetrahydrobenzyl) tetrahydrophthalate.

In the same equipment as described in Example 4, 25 moles (3,804.17 grams) of 1,2,3,6-tetrahydrophthalic anhydride and 65.4 moles (7334 grams) of tetrahydrobenzyl alcohol were added. Excess

tetrahydrobenzyl alcohol was used to facilitate the reaction. The reactor and its contents were swept with nitrogen. Then, with stirring, the system was heated to reflux with the thermowatch set at 230° C under a nitrogen atmosphere supplied by the bubbler system. Water of condensation was formed and removed by azeotropic distillation with tetrahydrobenzyl alcohol into the Dehn-Stark trap. About 5.25 hours after the heating was begun, approximately 395 mL of water of condensation had formed. At this time 2.2 grams of stannous octanoate, a catalyst for the condensation reaction, were added. The reaction was continued at reflux, 227° C overnight for a total reaction time of about 23.5 hours. The reaction mass was allowed to cool to room temperature. Determination of the acid number indicated a value of 0.25. Then the residual tetrahydrobenzyl alcohol was removed by distillation at 218° C. The resultant crude product was purified by distillation to yield 7,330.1 grams of the triene, bis(tetrahydrobenzyl) tetrahydrophthalate, with a platinum-cobalt color of 55. It was stored for future epoxidation.

Example 6. A 12-liter reactor equipped with a stirrer, temperature control device, pressure control, and feeding ports was purged with nitrogen and, with the purge continuing, 2,504 grams of bis(tetrahydrobenzyl) tetrahydrophthalate product of Example 5 and 150 grams of ethyl acetate were added. Stirring was begun and the reaction mass was heated to 25° C and held there. A vacuum sufficient to establish an ethyl acetate reflux but not greater than 30 mmHg was begun. Then, a ethyl acetate solution of peracetic acid, 22.65% peracetic acid, 6.36% acetic acid, and 70.99% ethyl acetate, was slowly fed to the reactor at a sufficiently low rate to keep the temperature between 22° C and 25° C. During the reaction, the vacuum was adjusted as necessary to control the reactor temperature between 22° C and 25° C. One hour after the peracetic acid was added, and every half-hour thereafter, the reaction mixture was analyzed by titration for peracetic acid content. When the peracetic acid content was constant, indicating the reaction was complete, a vacuum reflux was used to cool the reaction mixture to 0-5° C. The reaction mass was then flushed with 150 mL of ethyl acetate. Analysis indicated the reaction mass had the following composition:

| | |
|---|---|
| Epoxide of bis(tetrahydrobenzyl) tetrahydrophthalate | 25.82% |
| Bis(tetrahydrobenzyl) tetrahydrophthalate | 4.17% |
| Ethyl acetate | 50.56% |
| Acetic acid | 16.10% |
| Peracetic acid | 4.35% |

The ethyl acetate, peracetic acid, and acetic acid were then stripped from the reaction mass with a Pope evaporator. The distillate was collected in a 5-liter receiver that contained 1,740 grams of mixed xylenes that were at 0-5° C. The receiver was in an ice/water bath maintained at 0-5° C and the condenser temperature on the Pope evaporator was set at -15° C. Cold traps were in dry ice/acetone baths. The vacuum was set at 40 mmHg. After about one-half of the reaction mass had been fed, the receiver was removed and replaced with another 5-liter receiver that contained 1,740 grams of mixed xylenes that were at 0-5° C. The crude product was then washed three times with water to remove acetic acid, peracetic acid, and ethyl acetate with the decant portion discarded after each washing. The crude product, 5937.32 grams, contained 2490.11 grams of desired product, 2,561.77 grams of mixed xylenes, 3.22 grams of acetic acid/peracetic acid, 828.68 grams of ethyl acetate, and 53.54 grams of water. Xylenes and other volatiles were removed by stripping at 15-20° C and less than 10 mmHg. The triepoxide product comprising the compound

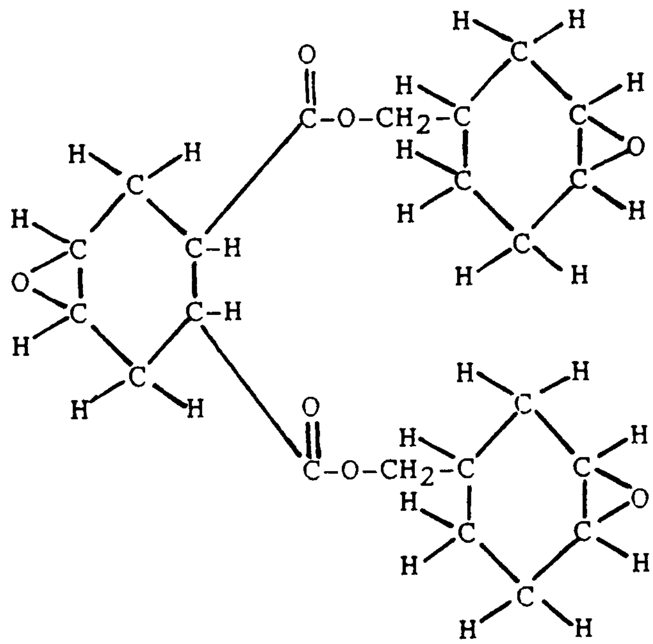

was stored for future use.

Examples 7-11. These examples describe the room temperature curing of the product of Example 6 with an acid terminated caprolactone polyol.

The following ingredients were placed in glass containers, well mixed, and applied to Bonderite 1000 steel panels with either a No. 6 or a No. 34 wire-wound rod. The coated panels were cured under room temperature, ambient conditions. The length of time required to obtain a tack-free surface was observed, and solvent resistance was measured by the methyl ethyl ketone double rub test.

| Ingredient, grams | EXAMPLE 7 | 8 | 9 | 10 | 11 |
|---|---|---|---|---|---|
| Example 6 Epoxide | 2.57 | 3.85 | 5.14 | 6.42 | 7.70 |
| Polyacid I | 5.00 | 5.00 | 5.00 | 5.00 | 5.00 |
| Methyl ethyl ketone | 4.08 | 4.77 | 5.46 | 6.15 | 6.84 |
| Surfactant | 0.06 | 0.07 | 0.08 | 0.09 | 0.10 |
| **Results with No. 6 Rod** | | | | | |
| Film Thickness, mils | 0.35 | 0.20 | 0.25 | 0.25 | |
| 0.20 | | | | | |
| Time to tack-free surface, hours | 5.5 | 4 | 4.5 | 4.5 | 5 |
| methyl ethyl double rubs after | | | | | |
| 1 day | None | None | None | None | None |
| 2 days | 1 | 1 | 1 | None | 1 |
| 4 days | 1 | 8 | 15 | 15 | 15 |
| 7 days | 1 | 10 | 20 | 40 | 25 |
| 10 days | 5 | 15 | 20 | 45 | 30 |

| | EXAMPLE 7 | 8 | 9 | 10 | 11 |
|---|---|---|---|---|---|
| Pencil hardness after | | | | | |
| 1 day | 4B | F | 6H | 4H | F |
| 2 days | 4B | F | H | H | H |
| 4 days | 2B | 3H | 5H | 3H | 4H |
| 7 days | B | 3H | 3H | 4H | 4H |

| Results with No. 34 Rod | | | | | |
|---|---|---|---|---|---|
| Film Thickness, mils | 1.15 | 1.25 | 1.35 | 1.25 | 1.25 |
| Time to tack-free surface, hours | 6 | 5.5 | 5.5 | 6 | 6 |
| methyl ethyl double rubs after | | | | | |
| 1 day | 3 | 3 | None | 2 | 2 |
| 2 days | 5 | 15 | 10 | 4 | 5 |
| 4 days | 7 | 100 | >100 | >100 | >100 |
| 7 days | 6 | >100 | -- | -- | -- |
| 10 days | 20 | -- | -- | -- | -- |
| Pencil hardness after | | | | | |
| 1 day | <6B | 6B | <6B | 5B | 6B |
| 2 days | <6B | 5B | 4B | 4B | 5B |
| 4 days | 2B | 2B | HB | F | F |
| 7 days | 2B | B | F | H | H |

These results indicate that the new triepoxide product can be used to prepare room temperature curable coatings that develop a tack-free surface quite soon after coating and good properties with the passage of time.

Example 12. The following example describes the thermal curing of the triepoxide composition of this invention with no attempt at determining the lowest cure temperature for the systems.

The following ingredients were placed in glass containers and coated onto an untreated steel substrate with a wire-wound rod. The coatings were then oven cured for 30 minutes at 120 ° C. Coating thickness was approximately 1 mil.

| Ingredient, parts per hundred parts | Control | EX. 12 |
|---|---|---|
| Epoxide of Example 6 | -- | 49.5 |
| 3,4-epoxycyclohexylmethyl 3,4-epoxycyclohexane carboxylate | 49.5 | -- |
| Methyl ethyl ketone | 50.0 | 50.0 |
| FC-520 | 0.5 | 0.5 |
| **Results** | | |
| Methyl ethyl ketone double rubs | >100 | >100 |
| Pencil Hardness | 2H | 3H |

The coating of Example 12 was hard and solvent resistance and had improved hardness over that of a diepoxide control.

Examples 13-17. Description of ultraviolet light curable coatings.

The following ingredients are placed in amber-colored glass bottles and stirred well. They are coated onto Bonderite No. 37 steel panels and onto heavy paper stock with a No. 20 wire-wound rod, and exposed to a 300 watt per inch medium pressure mercury vapor light source at 20 feet per minute. Smooth, glossy, attractive coatings result.

| | EXAMPLE | | | | |
|---|---|---|---|---|---|
| | 13 | 14 | 15 | 16 | 17 |
| Example 6 Epoxide | 50.0 | 30.0 | 40.0 | 10.0 | 65.0 |
| Cycloaliphatic Epoxide-1 | ---- | 37.5 | ---- | ---- | ---- |
| Cycloaliphatic Epoxide-2 | 10.0 | ---- | ---- | ---- | ---- |
| Cycloaliphatic Epoxide-3 | 25.5 | 15.0 | 54.5 | 76.5 | 16.0 |
| Epoxide-4 | ---- | ---- | ---- | ---- | 3.0 |
| Polyol-1 | ---- | 15.0 | ---- | ---- | 7.0 |
| Polyol-2 | 10.0 | ---- | ---- | ---- | ---- |
| Polyol-3 | ---- | ---- | ---- | 12.0 | ---- |
| Triethylene glycol Divinylether | ---- | ---- | 3.0 | ---- | 5.0 |
| Photoinitiator-1 | ---- | 2.0 | 2.0 | 1.0 | 1.5 |
| Photoinitiator-2 | 4.0 | ---- | ---- | ---- | 2.0 |
| Surfactant | 0.5 | 0.5 | 0.5 | 0.5 | 0.5 |

Examples 18-20. Description of thermally cured coatings containing trifunctional cycloaliphatic epoxide compositions.

The following ingredients are mixed in suitable glass or metal containers, coated onto tin plate and steel panels, and cured in a forced air oven. The coatings of Example 18-20 are baked at 120 °C for 20 minutes. Smooth, glossy, and adherent coatings result.

| | EXAMPLE | | |
|---|---|---|---|
| | 18 | 19 | 20 |
| Example 6 Epoxide | 65.0 | 30.0 | 10.0 |
| Cycloaliphatic Epoxide-1 | ---- | 60.0 | 50.0 |
| Cycloaliphatic Epoxide-2 | ---- | ---- | 20.0 |
| Cycloaliphatic Epoxide-3 | 25.0 | ---- | ---- |
| Polyol-1 | 9.0 | ---- | 10.0 |
| Triethylene glycol Divinylether | ---- | 9.0 | 9.0 |
| FC-520 | 0.5 | 0.3 | 0.5 |
| Surfactant | 0.5 | 0.5 | 0.5 |
| Methyl Amyl Ketone | 20.0 | 25.0 | 25.0 |

## Claims

**1.** A trifunctional unsaturated compound of the formula:

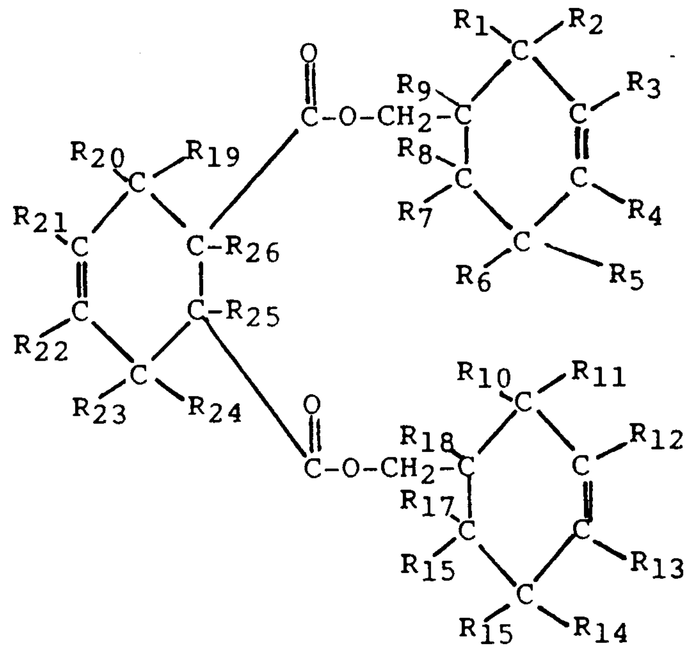

I

wherein $R_1$ to $R_{26}$ are the same or different and are hydrogen, phenyl, or unsubstituted or substituted alkyl or cycloalkyl groups of one to about 8 carbon atoms.

**2.** A trifunctional unsaturated compound as in Claim 1 wherein $R_1$ to $R_{26}$ are the same or different and are hydrogen, phenyl, or unsubstituted or substituted alkyl or cycloalkyl groups of one or two carbon atoms.

**3.** A trifunctional unsaturated compound as in Claim 1 wherein $R_3$ and $R_{12}$ are methyl and $R_1$, $R_2$, $R_4$ to $R_{11}$, and $R_{13}$ to $R_{26}$ are hydrogen.

**4.** A trifunctional unsaturated compound as in Claim 1 wherein $R_4$ and $R_{13}$ are methyl and $R_1$ to $R_3$, $R_5$ to $R_{12}$, and $R_{14}$ to $R_{26}$ are hydrogen.

**5.** A trifunctional unsaturated compound as in Claim 1 wherein $R_7$ and $R_{15}$ are methyl and $R_1$ to $R_6$, $R_8$ to $R_{14}$, and $R_{16}$ to $R_{26}$ are hydrogen.

**6.** A trifunctional unsaturated compound as in Claim 5 wherein $R_3$ and $R_{12}$ are methyl.

**7.** A trifunctional unsaturated compound as in Claim 5 wherein $R_4$ and $R_{13}$ are methyl.

**8.** A trifunctional unsaturated compound as in Claim 1 wherein $R_1$ to $R_{26}$ are hydrogen.

**9.** A trifunctional cycloaliphatic epoxide compound comprising the epoxidized derivative of the trifunctional unsaturated compound of Claim 1 with the following formula:

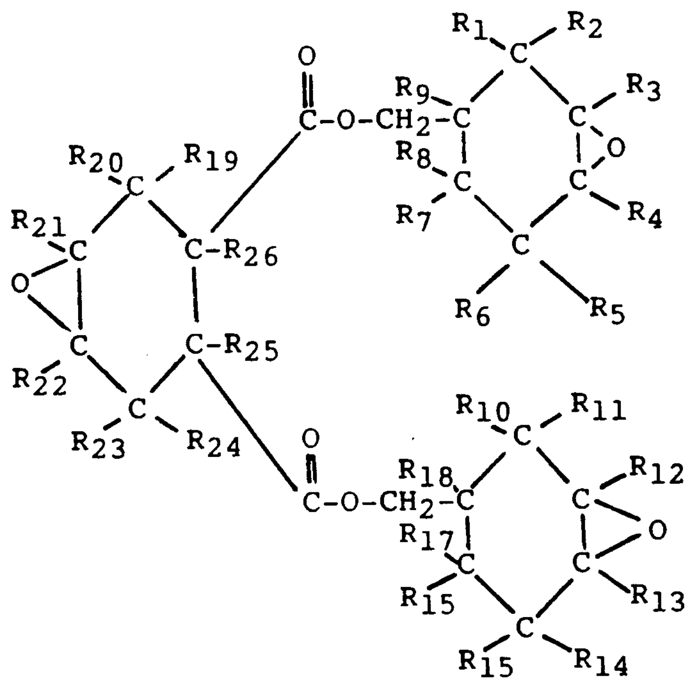

10. A trifunctional cycloaliphatic epoxide compound as in Claim 9 wherein $R_1$ to $R_{26}$ are the same or different and are hydrogen, phenyl, or unsubstituted or substituted alkyl or cycloalkyl groups of one or two carbon atoms.

11. A trifunctional cycloaliphatic epoxide compound as in Claim 9 wherein $R_3$ and $R_{12}$ are methyl and $R_1$, $R_2$, $R_4$ to $R_{11}$, and $R_{13}$ to $R_{26}$ are hydrogen.

12. A trifunctional cycloaliphatic epoxide compound as in Claim 9 wherein $R_4$ and $R_{13}$ are methyl and $R_1$ to $R_3$, $R_5$ to $R_{12}$, and $R_{14}$ to $R_{26}$ are hydrogen.

13. A trifunctional cycloaliphatic epoxide compound as in Claim 9 wherein $R_7$ and $R_{15}$ are methyl and $R_1$ to $R_6$, $R_8$ to $R_{14}$, and $R_{16}$ to $R_{26}$ are hydrogen or methyl.

14. A trifunctional cycloaliphatic epoxide compound as in Claim 13 wherein $R_3$ and $R_{12}$ are methyl.

15. A trifunctional cycloaliphatic epoxide compound as in Claim 13 wherein $R_4$ and $R_{13}$ are methyl.

16. A trifunctional cycloaliphatic epoxide compound as in Claim 9 wherein $R_1$ to $R_{26}$ are hydrogen.

17. A thermally curable coating composition comprising:
(a) 1 to about 99 weight percent of a trifunctional cycloaliphatic epoxide compound of Claim 9,
(b) 0 to 75 weight percent of a polyacid,
(c) 0 to 95 weight percent of a difunctional cycloaliphatic epoxide,
(d) 0 to 50 weight percent of a monoepoxyfunctional cycloaliphatic epoxide,
(e) 0 to 60 weight percent of a polyol,
(f) 0 to 3 weight percent of a catalyst, and
(g) optionally a surfactant and/or an inert solvent.

18. A composition as in Claim 17 wherein component (f) is from 0.05 to 3 weight percent.

19. A composition as in Claim 17 wherein the trifunctional cycloaliphatic epoxide compound is of the following formula:

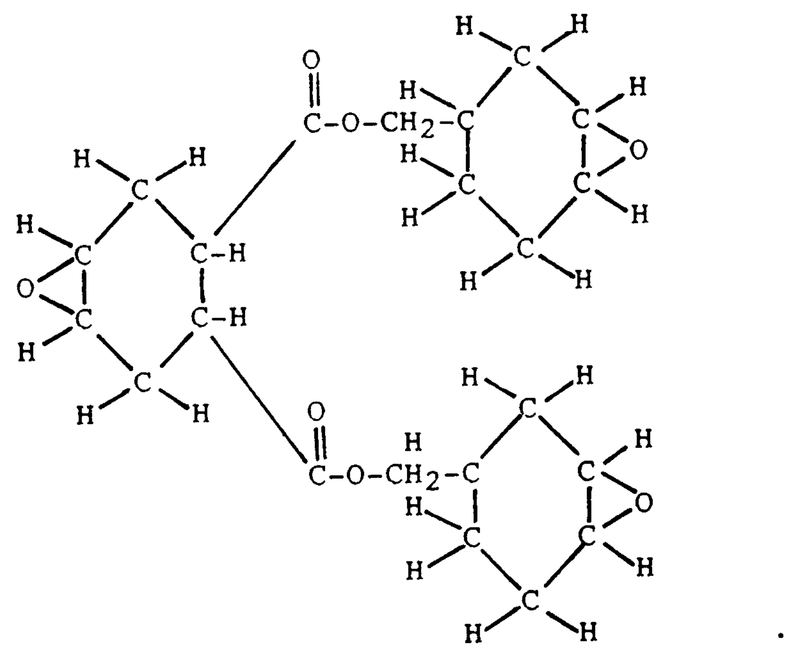

20. A composition as in Claim 17 wherein the difunctional cycloaliphatic epoxide is 3,4-epoxycyclohexylmethyl 3,4-epoxycyclohexanecarboxylate.

21. A composition as in Claim 17 wherein the polyacid is a polycaprolactone polyol end capped with phthalic anhydride.

22. A composition as in Claim 21 wherein the polyol is end-capped with maleic anhydride.

23. A composition as in Claims 21 or 22 wherein the molecular weight of the polycaprolactone polyol is from about 300 to about 2000.

24. A photocurable coating composition comprising:
   (a) 1 to about 99 weight percent of a trifunctional cycloaliphatic epoxide compound of Claim 9,
   (b) 0 to 95 weight percent of a difunctional cycloaliphatic epoxide,
   (c) 0 to 50 weight percent of a monoepoxyfunctional cycloaliphatic epoxide,
   (d) 0 to 60 weight percent of a polyol,
   (e) 0.05 to 10 weight percent of an onium salt photoinitiator,
   (f) 0 to about 35% of a monoacrylate or polyfunctional acrylate, and
   (g) optionally a surfactant, free-radical generating photoinitiator, and/or inert solvent.

25. A composition as in Claim 24 wherein component (e) is from 0.10 to 5 weight percent.

26. A composition as in Claim 24 wherein the trifunctional cycloaliphatic epoxide compound is of the following formula:

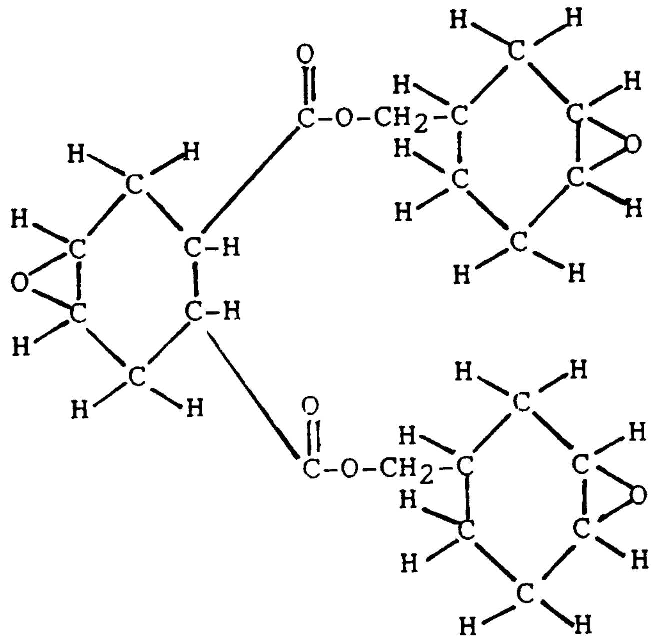

.

**27.** A composition as in Claim 24 wherein the difunctional cycloaliphatic epoxide is 3,4-epoxycyclohexylmethyl 3,4-epoxycyclohexanecarboxylate.

**28.** A composition as in Claim 27 wherein the polyol is a polycaprolactone polyol.

**29.** A composition as in Claim 27 wherein the polyol is a polyether polyol.

**30.** A cured film obtained from the composition of Claim 17 or 24.

**31.** A trifunctional unsaturated compound of Claim 1 comprising the reaction product of an anhydride of the formula:

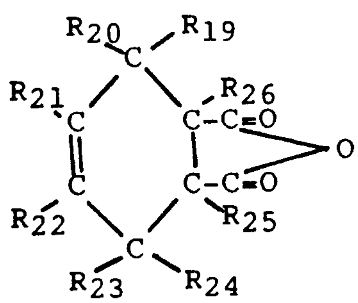

III

with an unsaturated alcohol of structural formulas:

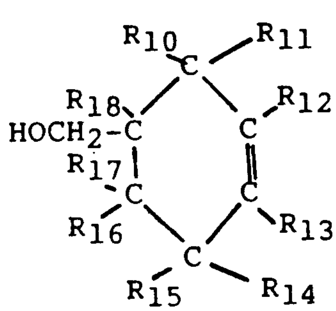

IV

and

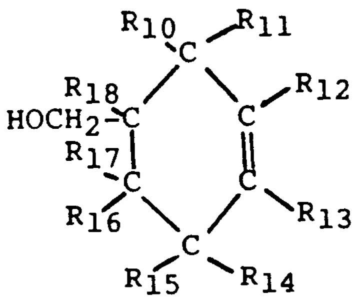

V

wherein $R_1$ to $R_{26}$ are the same or different and are hydrogen, phenyl, or unsubstituted or substituted alkyl or cycloalkyl groups of one to about 8 carbon atoms.

**32.** A trifunctional unsaturated compound as in Claim 31 wherein $R_3$ and $R_{12}$ are methyl and $R_1$, $R_2$, $R_4$ to $R_{11}$, and $R_{13}$ to $R_{26}$ are hydrogen.

**33.** A trifunctional unsaturated compound as in Claim 31 wherein $R_4$ and $R_{13}$ are methyl and $R_1$ to $R_3$, $R_5$ to $R_{12}$, and $R_{14}$ to $R_{26}$ are hydrogen.

**34.** A trifunctional unsaturated compound as in Claim 31 wherein $R_1$ to $R_{26}$ are hydrogen.

**35.** A product comprising the epoxidized product of Claim 31.

**36.** A method for producing the product of Claim 35 wherein the alcohol and anhydride are:
(a) reacted at a temperature of 100 to 300 °C for 2 to 72 hours in the presence of a catalyst,
(b) the resulting product is refined by washing with water, evaporation, filtration, decolorized, and/or distillation,
(c) the resulting product is reacted with an oxidizing agent at a temperature of about 5 to 90 °C, and
(d) purified by neutralization, evaporation, and/or distillation.

**37.** A method as in Claim 36 wherein the product is decolorized by:
(a) dissolving in a solvent or mixture of solvents, and
(b) passing through a filtration bed composed of activated charcoal and/or activated alumina.

**38.** A method as in Claim 36 wherein the catalyst is stannous oxalate.

**39.** A method as in Claim 36 wherein the catalyst is stannous octanoate.

**40.** A method as in Claim 36 wherein the oxidizing agent is peracetic acid.